# EUROPÄISCHE PATENTSCHRIFT

(11) **EP 1 007 021 B1**
(45) Veröffentlichungstag und Bekanntmachung des Hinweises auf die Patenterteilung: **13.06.2001**
(21) Anmeldenummer: 98908080.9
(22) Anmeldetag: 11.02.1998
(51) Int. Cl.: A61K 31/12, A61K 47/00

(54) **TRANSDERMALE, ORALE UND INTRAVENÖSE ZUBEREITUNGEN VON 2,3-DIMETHOXY-5-METHYL-6-DECAPRENYL-1,4-BENZOCHINON**
TRANSDERMAL, ORAL AND INTRAVENOUS PREPARATIONS OF 2,3-DIMETHOXY-5-METHYL-6-DECAPRENYL-1,4-BENZOQUINONE
PREPARATIONS TRANSDERMIQUES, ORALES ET INTRAVEINEUSES DE 2,3-DIMETHOXY-5-METHYLE-6-DECAPRENYLE-1,4-BENZOQUINONE

(30) Priorität: 12.02.1997 DE 19705231
(43) Veröffentlichungstag der Anmeldung: 14.06.2000
(73) Patentinhaber: MSE Pharmazeutika GmbH, 61348 Bad Homburg (DE)
(72) Erfinder: ENZMANN, Franz, D-61348 Bad Homburg (DE); LACHMANN, Burkhard, NL-3065 BC Rotterdam (NL)
(74) Vertreter: Werner, Hans-Karsten, Dr.Dipl.-Chem.
(86) Internationale Anmeldenummer: EP9800744
(87) Internationale Veröffentlichungsnummer: WO9835660

(56) Entgegenhaltungen:
- EP-A- 0 335 133
- EP-A- 0 461 333
- WO-A-90/07469
- WO-A-96/13254
- DE-A- 4 327 063
- US-A- 5 451 569

## Beschreibung

2,3-Dimethoxy-5-methyl-6-decaprenyl-1,4-benzochinon ist auch bekannt unter der Bezeichnung Coenzym Q-10. Die Substanz spielt eine Rolle in der Atmungskette und ist obendrein ein Antioxidanz, welches in der Lage ist, Radikale, die insbesondere von Vitaminen weitergegeben werden, abzufangen und unschädlich zu machen. Q-10 bestimmt außerdem die Elastizität und die Dynamik der Zellmembranen. Es wird daher als Monopräparat und in Kombination mit anderen Wirkstoffen zur oralen Einnahme empfohlen. Zur Hautpflege wird es darüber hinaus in Form einer Liposomencreme angeboten, welche es dem Wirkstoff gestattet, durch die Hornschichtbarrieren einzudringen und sich dann in den verschiedenen Schichten der Haut anzureichern. Die bisher verwendete Liposomcreme wird hergestellt auf Basis von Lecithinen und bildet eine Lipiddoppelschicht um einen wäßrigen Innenraum. Q-10 lagert sich dabei direkt innerhalb der Membran an.

Es wurde jetzt gefunden, daß transdermale, orale und intravenöse Zubereitungen von 2,3-Dimethoxy-5-methyl-6-decaprenyl-1,4-benzochinon verbessert und wirksamer gemacht werden können, wenn sie außer den üblichen Hilfsstoffen eine wirksame Menge des Pulmonary Surfactant enthalten. Der Pulmonary Surfactant ist ein Komplex von Phospholipiden, neutralen Lipiden und Surfactant Proteinen, die miteinander eine einschichtige Barriere zwischen der Luft und der flüssigen Oberfläche der Lunge ausbilden. Der Pulmonary Surfactant wird in den Alveolaren Typ II Zellen gebildet und von dort in den Alveolarzwischenraum abgegeben.

Da der Pulmonary Surfactant von den Alveolaren Typ II Zellen in den Luftraum der Lungen abgegeben wird, wurde nicht daran gedacht, daß Pulmonary Surfactant in Gewebsschichten eindringen würde. Der Pulmonary Surfactant ist daher bisher nur eingesetzt worden zur Instillation bei Erkrankungen oder Mangelerscheinungen der Lunge und zum Transport von Antibiotika und Corticosteroiden in die Lunge.

Andere Anwendungen sind bisher nicht in Erwägung gezogen worden. Es wurde nun gefunden, daß Pulmonary Surfactant unerwarteterweise in die äußere Haut und die Schleimhaut des Magen-Darmbereichs, des Mund- und Vaginalbereichs einzudringen vermag und zwar allein oder in Zusammenwirkung mit Liposomen.

Dabei spielt es eine untergeordnete Rolle, ob hochgereinigte oder weniger gereinigte Pulmonary Surfactant-Präparationen aller möglichen Spezies oder durch Rekombination gewonnene Pulmonary Surfactant herangezogen werden (Schwein, Rind, Schaf etc.). Weniger gereinigte Präparationen haben den Vorteil, daß sie sich kostengünstig produzieren lassen.

Da jegliches überbeanspruchte Gewebe einen mehr oder weniger Q-10 Mangel aufweist, wurde probiert, Q-10 mit Hilfe des Pulmonary Surfactant in die unterversorgten Bereiche zu transportieren. Eine Kombination von Liposomen und Pulmonary Surfactant erwies sich tatsächlich als vorteilhaft.

Die erfindungsgemäße Zubereitung enthaltend 2,3-Dimethoxy-5-methyl-6-decaprenyl-1,4-benzochinon und eine wirksame Menge des Pulmonary Surfactant kann somit mit sehr gutem Erfolg eingesetzt werden zur oralen Behandlung von Krankheiten des Herz-Kreislaufs, der Lunge, der Muskulatur, von Magen und Darm (Ulcus und Gastritis), Diabetes, der Haut, der Nerven, Tinnitus, bei degenerativen Stoffwechselentgleisungen, Inkontinenz, Paradontose, mitochondrialen Erkrankungen, Immunschwäche und Rheuma.

Darüber hinaus wurde festgestellt, daß diese erfindungsgemäße Kombination auch erfolgreich eingesetzt werden kann zur topischen Behandlung von Psoriaris, Neurodermitis, Verbrennungen, Strahlenschäden, Ekzemen, Wunden, Ulcus cruris, Hautkrebs und Hautalterung.

## Patentansprüche

1. Transdermale, orale oder intravenöse Zubereitung von 2,3-Dimethoxy-5-methyl-6-decaprenyl-1,4-benzochinon enthaltend außer üblichen Hilfsstoffen eine wirksame Menge des Pulmonary Surfactant.

2. Zubereitung gemäß Anspruch 1, dadurch gekennzeichnet, daß die Zubereitung in Kombination mit Liposomen eingesetzt wird.

3. Zubereitung gemäß Anspruch 1 oder 2, dadurch gekennzeichnet, daß der Pulmonary Surfactant als Rohextrakt eingesetzt wird.

4. Zubereitung gemäß Anspruch 1 bis 3 zur oralen Behandlung von Krankheiten des Herz-Kreislaufs, der Lunge, Muskulatur, von Magen und Darm (Ulcus und Gastritis), Diabetes, der Haut, der Nerven, Tinnitus, bei degenerativen Stoffwechselentgleisungen, Inkontinenz, Paradontose, mitochondrialen Erkrankungen, Immunschwäche und Rheuma.

5. Zubereitung gemäß Anspruch 1 zur topischen Behandlung von Psoriaris, Neurodermitis, Verbrennungen, Strahlenschäden, Ekzemen, Wunden, Ulcus cruris, Hautkrebs, Hautalterung.

## Claims

1. A transdermal, oral or intravenous formulation of 2,3-dimethoxy-5-methyl-6-decaprenyl-1,4-benzoquinone containing an effective amount of pulmonary surfactant in addition to usual auxiliaries.

2. The formulation according to claim 1, characterized by being employed in combination with liposomes.

3. The formulation according to claim 1 or 2, characterized in that said pulmonary surfactant is used as a raw extract.

4. The formulation according to claims 1 to 3 for the oral treatment of diseases of the cardiovascular system, the lungs, muscles, stomach and intestine (ulcer and gastritis), diabetes, diseases of the skin, the nerves, tinnitus, degenerative metabolic imbalance, incontinence, periodontosis, mitochondrial diseases, immunodeficiency and rheumatism.

5. The formulation according to claim 1 for the topical treatment of psoriasis, neurodermitis, burns, radiation injuries, eczemas, wounds, ulcus cruris, cancer of the skin, skin ageing.

## Revendications

1. Préparation transdermique, orale ou intraveineuse de 2,3-diméthoxy-5-méthyl-6-décaprényl-1,4-benzoquinone contenant, outre des adjuvants usuels, une quantité efficace de surfactant pulmonaire.

2. Préparation selon la revendication 1, caractérisée en ce que la préparation est utilisée en association avec des liposomes.

3. Préparation selon la revendication 1 ou 2, caractérisée en ce que le surfactant pulmonaire est utilisé sous forme d'extrait brut.

4. Préparation selon les revendications 1 à 3 pour le traitement oral de maladies du système cardiovasculaire, des poumons, des muscles, de l'estomac et de l'intestin (ulcère et gastrite), du diabète, de la peau, des nerfs, du tintement, lors des troubles dégénératifs du métabolisme, de l'incontinence, de la parodontose, des maladies mitochondriales, de la faiblesse immunitaire et du rhumatisme.

5. Préparation selon la revendication 1 pour le traitement topique du psoriasis, de la névrodermite, des brûlures, des lésions dues à des rayonnements, des eczémas, des blessures, de l'ulcère variqueux, du cancer de la peau, du vieillissement de la peau.
